(19) Europäisches Patentamt
European Patent Office
Office européen des brevets

(11) Publication number: **0 144 960**
**A2**

# (12) EUROPEAN PATENT APPLICATION

(21) Application number: 84114701.0

(22) Date of filing: 03.12.84

(51) Int. Cl.[4]: A 61 K 7/22
C 07 D 275/06, C 07 D 401/06

(30) Priority: 09.12.83 US 559997
09.12.83 US 560059

(43) Date of publication of application:
19.06.85 Bulletin 85/25

(84) Designated Contracting States:
AT BE CH DE FR GB IT LI NL SE

(71) Applicant: STERLING DRUG INC.
90 Park Avenue
New York New York(US)

(72) Inventor: Alford, Charles Edmund
491 Haskell Place
Delmar New York(US)

(72) Inventor: Gorman, William George
25 Old Troy Road
East Greenbush New York(US)

(72) Inventor: Mavica, Kenneth Michael
11 Muirfield Lane
Clifton Park New York(US)

(72) Inventor: Popp, Karl Frederick
Duck Pond Road
Schodack Landing New York(US)

(74) Representative: Baillie, Iain Cameron et al,
c/o Ladas & Parry Isartorplatz 5
D-8000 München 2(DE)

## (54) Dentrifice compositions and novel saccharine salts and preparation thereof.

(57) Antiplaque dentifrices containing as the antiplaque ingredient the saccharin salt of an amino or quaternary ammonium antimicrobial agent, particularly chlorhexidine saccharin salt or octenidine saccharin salt, and the method of inhibiting, reducing and preventing dental plaque therewith are disclosed. Novel bis[4-(substituted-amino)-1-pyridinium]alkane saccharin salts are prepared from corresponding relatively soluble salts and saccharin or a soluble salt of saccharin and are useful as antimicrobial agents.

EP 0 144 960 A2

0144960

## BACKGROUND OF THE INVENTION

### Field of the Invention

The invention relates to dentifrice compositions containing an antiplaque ingredient. The invention also relates to novel bis[4-(substituted-amino)-pyridinium] alkane saccharin salts which are useful as antimicrobial agents and a process for preparation thereof.

U.S. Pat. 2,684,924 issued July 27, 1954 describes antimicrobial aryl bisbiguanides including chlorhexidine (The Merck Index, Ninth Edition, 1976, monograph 2060).

Chemical Abstracts (vol. 59, p. 11333b, 1963) sets forth an abstract of Japanese Patent 4891 (1963) describing bis(p-chlorophenylbiguanido)hexane saccharinate, which is the saccharin salt of chlorhexidine.

U.S. Pat. 4,053,636 issued October 11, 1977 describes antimicrobial dichlorocyclopropylphenyl bisbiguanides.

U.S. Pat. 3,468,898 issued September 23, 1969 describes antimicrobial alkyl bisbiguanides including alexidine, which is shown by The Merck Index cited above (monograph 224).

U.S. Pat. 4,022,834 issued May 10, 1977 describes antimicrobial cycloalkyl bisbiguanides.

Petrocci (chapter entitled "Quaternary Ammonium Compounds" in Disinfection, Sterlization, and Preservation, Seymour S. Block, Editor, 2nd Edition, Lea & Febiger, Philadelphia, 1977, pp. 325-347) describes the quaternary ammonium disinfectants, which are a well-known class of antimicrobial agents. Particularly well-known examples

shown by The Merck Index cited above are benzalkonium chloride (monograph 1059), benzethonium chloride (monograph 1078), cetylpyridinium chloride (monograph 1987) and dequalinium chloride (monograph 2874).

U.S.Pat. 4,206,215 issued June 3, 1980 describes antimicrobial bis[4-(substituted-amino)-1-pyridinium] alkane salts and the dental plaque preventing method of use of certain species thereof. U.S. Pat. 3,538,230 issued November 3, 1970 describes dentifrices containing silica erogels as cleaning and polishing agents. U.S. Pat. 4,303,641 issued December 1, 1981 describes dentifrices containing hydrous silica gels as cleaning and polishing agent. U.S. Pat. 2,689,170 issued September 14, 1954 describes dentifrices containing higher alkanoylamino-alkanecarboxylic acids and salts thereof, especially sodium N-lauroyl sacrcoside (sarcosinate) as caries inhibiting agents. Monograph 4205 of The Merck Index cited above describes sodium N-lauroyl sarcosinate (Gardol® as being useful as a detergent, foaming agent and antienzyme for dentifrices.

Parts A and B of Example 10 of above-cited U.S. Pat. 4,206,215 describe 1,10-bis[4-(octylamino)-1-pyridinium]decane dichloride. The same compound can also be described by the name N,N'(1,10-decanediyldi-1(4H)-pyridyl-4-ylidene)bis[1-octaneamine] dihydrochloride, whose hypothetical free base form has been given the generic name octenidine. In oral use the bis[4-(substituted-amino)-1-pyridinium]alkane salts subgenerically and specifically described by U.S. Pat. 4,206,215, for example, the dihydrochloride salt of octenidine, have an extremely bitter taste and a lingering bitter metallic aftertaste.

Attempts have been made to formulate antiplaque dentifrices and one such product is CORSODYL™ Dental GEL containing 1% by weight of chlorhexidine gluconate (manufactured by Imperial Chemical Industries Limited, Pharmaceuticals Division, Macclesfield, Cheshire, England), but these attempts including CORSODYL™ Dental Gel have

been generally unsuccessful due to the problem of combining antiplaque activity, good taste, good foaming ability and good polishing ability in a single formulation. The presently described and claimed invention overcomes this problem and provides such a formulation.

In one aspect the invention deals with an antiplaque dentifrice comprising

A) an effective dental plaque-inhibiting, -reducing or -preventing amount of the saccharin salt of an amino or quaternary ammonium antimicrobial agent which is

a) a compound having the structural formula

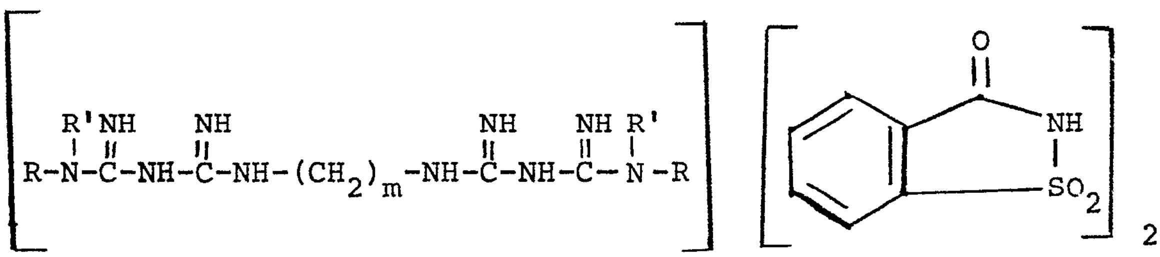

Formula I

wherein R taken alone is phenyl substituted by alkyl, alkoxy, nitro or halo, p-(2,2-dichlorocyclopropyl)phenyl, alkyl having from 6 to 16 carbon atoms, cycloalkyl or polycyclic alkyl having 5 or more carbon atoms or lower-alkyl-cycloalkyl or cycloalkyl-lower-alkyl having from 1 to 4 carbon atoms in lower alkyl; R' taken alone is hydrogen; R and R' taken together are 3-azabicyclo (3,2,2)nonyl; and n is an integer from 3 to 9;

b) a compound having the structural formula

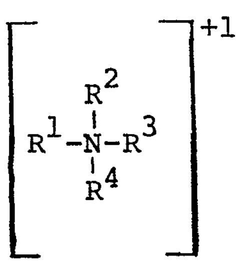

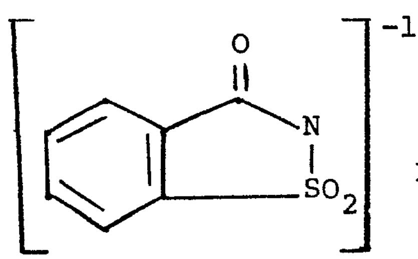

Formula II or

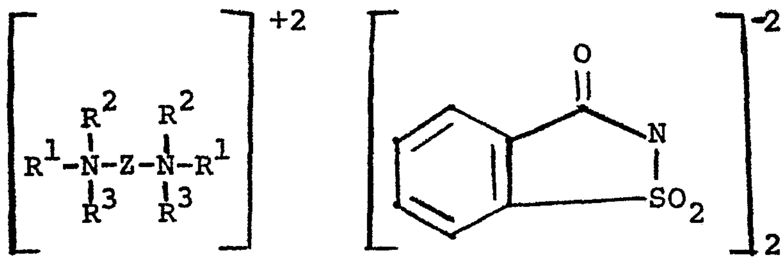

Formula III

wherein $R^1$ is long-chain alkyl or aralkyl; $R^2$ is short-chain alkyl, long-chain alkyl or aralkyl, benzyl or part of an aromatic system or non-aromatic system; $R^3$ and $R^4$ are short-chain alkyl or part of an aromatic ring system or non-aromatic ring system; and Z is a carbon-hydrogen chain; or

c) a compound having the structural formula

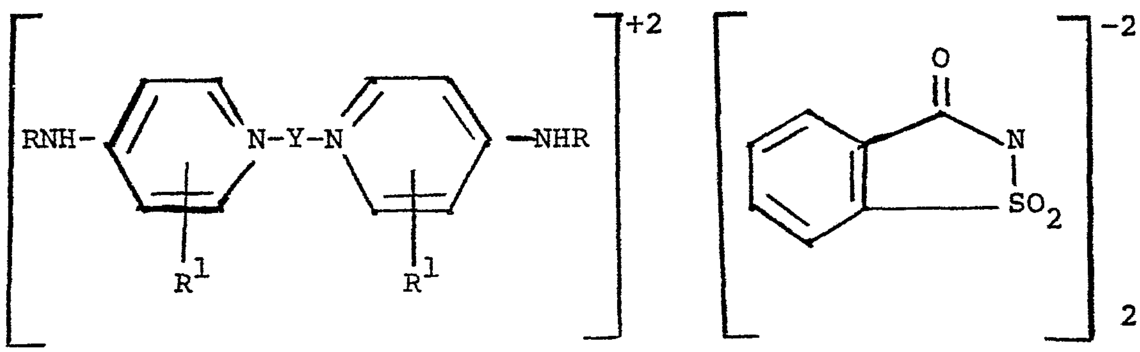

Formula IV

wherein Y is alkylene containing from 4 to 18 carbon atoms and separating the two 4-(R-amino)-1-pyridinium groups by from 4 to 18 carbon atoms; R is the same in both occurrences and is alkyl containing from 6 to 18 carbon atoms, cycloalkyl containing from 5 to 7 carbon atoms, benzyl or phenyl substituted by methylenedioxy or one or two substituents selected from the group consisting of halo, lower-alkyl, lower alkoxy, nitro, cyano and trifluoromethyl; and $R_1$ is the same in both occurrences and is hydrogen

or lower-alkyl;

B) a polishing agent or thickening agent which is silicon dioxide, silicic acid, silica, amorphous silica, precipitated silica, hydrated silica, silica hydrate, silica gel, silica xerogel or hydrous silica gel; and

C) a foaming agent which is N-alkanoylsarcosine or N-alkenoylsarcosine or an alkali metal, ammonium or alkanolamine salt thereof, wherein alkanoyl or alkenoyl has from 8 to 18 carbon atoms or alkanolamine has from 2 to 9 carbon atoms.

The compounds of Formula IV as defined above are novel and represent a further aspect of the invention.

The compounds of Formula IV can also be described as N,N'(alkanediyldi-1(4H)-pyridin-4-ylidene)bis[1-R-amino] disaccharin salts having the structural formula

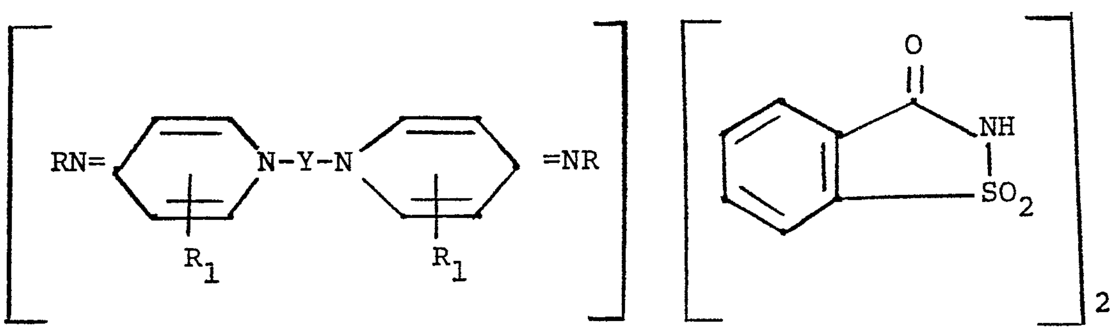

Formula V

wherein Y, R and $R_1$ have the same meanings set forth above for Formula IV. The compounds of Formula IV or V are useful as antimicrobial agents and are particularly useful as agents for inhibiting, reducing and preventing dental plaque in dentifrices.

One can inhibit, reduce or prevent dental plaque by contacting the dental plaque itself or the natural or artificial teeth or oral cavity of a living person with an effective dental plaque-inhibiting, -reducing or -preventing amount of the above-described antiplaque dentifrice.

The non-saccharin salts corresponding to the

compounds of Formulas I-IV are generally and particularly described by the above-cited prior art, which in some instances also describes the saccharin salts. Those saccharin salts of Formulas I-IV which are not described by the prior art are prepared by reacting in an aqueous solvent a corresponding relatively soluble non-saccharin salt with saccharin or a soluble salt of saccharin and separating the relatively insoluble saccharin salt.

The novel compounds of Formula IV can be prepared by reacting in an aqueous solvent a corresponding relatively soluble bis[4-(R-amino)-L-pyridinium]alkane non-saccharin salt with saccharin or a soluble salt of saccharin and separating the relatively insoluble bis[4-(R-amino)-1-pyridinium]alkane disaccharin salt.

The aqueous solvent can be water alone, which is the preferred solvent, or a mixture of water and one or more water-miscible organic solvents selected from the alcohols, ketones, acids, amides, nitriles and sulfoxides and multifunctional and mixed functional solvents. Since saccharin is only slightly soluble in water, a soluble salt, for example, an alkali metal salt, is preferably used. Saccharin sodium salt is most preferred. The reaction is preferably carried out at a temperature in the range of 0-100°C., most preferably 20-60°C.

A preferred saccharin salt of Formulas I-IV is the compound of Formula I wherein R is p-chlorophenyl, R' is hydrogen and n is 6, which is the saccharin salt of chlorhexidine and is described by the above-cited Chemical Abstracts abstract of Japanese Patent 4891. Another preferred saccharin salt of Formulas I-IV is the compound of Formula IV wherein R is octyl, $R_1$ is hydrogen and Y is 1,10-decylene, which is the saccharin salt of octenidine and is prepared from molar equivalent quantities of octenidine dihydrochloride salt and saccharin sodium salt in water (m.p. 82°C.).

The effective dental plaque-inhibiting, reducing or preventing amount of the saccharin salt of Formulas I-IV is generally in the range of 0.1-10% and most generally

of the order of 1% by weight of the dentifrice.

Silicic acid, silica, amorphous silica, precipitated silica, hydrated silica, silica hydrate, silica gel, silica xerogel and hydrous silica gel are all forms of silicon dioxide ($SiO_2$) differing in the method of formation and extent of hydration. Of these hydrous silica gel (hydrated silica) is most preferred. Different grades of hydrated silica are available for polishing and thickening. The dentifrices of the invention preferably contain both a polishing grade and a thickening grade of hydrated silica. The amount of polishing agent and thickening agent is generally in the range of 5-50% by weight of the dentifrice.

In N-alkanoylsarcosine and N-alkenoylsarcosine alkanoyl and alkenoyl having from 8 to 18 carbon atoms can be branched or unbranched. Unbranched alkanoyl having an even number of carbon atoms is preferred, especially lauroyl having 12 carbon atoms. Preferred alkanolamine salts include the monoethanolamine, diethanolamine and triethanolamine salts. The alkali metal salts include the lithium, sodium, and potassium salts. Sodium salts are preferred. The most preferred foaming agent is sodium N-lauroyl sarcosinate. The amount of foaming agent is generally in the range of 0.1-10% and most generally of the order of 1% by weight of the dentifrice.

In addition to the above-described components the dentifrices of the invention generally also contain one or more humectants, for example, sorbitol, glycerin or a polyethylene glycol, for example, PEG-6-32, which is an adopted name for a mixture of the polyethylene glycol having 6 ethylene oxide units and the polyethylene glycol having 32 ethylene glycol units; caries reducing, inhibiting or preventing fluoride salts, for example, sodium fluoride, sodium monofluorophosphate or stannous fluoride; sweeteners, for example, free saccharin in addition to the bound saccharin of the above-described saccharin salts, cyclamate or aspartame; flavors; colorants; acids or bases to adjust the pH (desirably about 7), for example, hydrochloric acid or sodium hydroxide; and water.

The following is an example of the preparation of a novel compound of Formula IV.

EXAMPLE I

A warm (50°C.) solution of N,N'-(1,10-decanediyldi-1(4H)-pyridin-4-ylidene)bis[1-octanamine](octenidine) dihydrochloride salt (623 g., 1.00 mole) in deionized water (6 l.) was added with stirring during 15 minutes to a warm (50°C.) solution of saccharin sodium salt (520 g., 2.00 mole) in deionized water (2 l.). Stirring was continued for two hours, then the mixture was filtered. The solid was washed with hot deionized water (3 l.) and dried at 60°C. under vacuum, affording N,N'-(1,10-decanediyldi-1(4H)-pyridin-4-ylidene)bis[1-octanamine] (octenidine) disaccharin (saccharin)salt (molecular weight 917.1, 890 g., 97% yield, m.r. 82-85°C.).

In preparing the dentifrices of the invention the saccharin salts of Formulas I-IV can be formed *in situ*, but use of preformed saccharin salt is preferred. To ensure maximum possible antiplaque effect the preformed saccharin salt is added as the last ingredient of the dentifrice formulation. A typical order of addition of the remaining ingredients of a typical formulation is: water, fluoride salt, humectants, sweetener, foaming agent, flavors, colorant, acid or base to adjust pH, and polishing and thickening agents. Accordingly the following examples were prepared.

Example 2

| Ingredient | Percent by Weight |
| --- | --- |
| Chlorhexidine Saccharin Salt | 1.10 |
| Sodium Fluoride | 0.220 |
| Sorbitol Solution | 46.3 |
| Hydrated Silica, Polishing Grade | 17.0 |
| Gylcerin | 15.0 |
| Hydrated Silica, Thickening Grade | 11.1 |
| PEG-6-32 | 3.00 |
| Sodium Lauroyl Sarcosinate | 2.10 |
| Flavors | 1.100 |
| Saccharin | 0.100 |
| Colorants | 0.002500 |
| Hydrochloric Acid to make pH 7 | -- |
| Purified Water to make | 100.0 |

Example 3

| Ingredient | Percent by Weight |
| --- | --- |
| Octenidine Saccharin Salt | 1.10 |
| Sodium Fluoride | 0.220 |
| Sorbitol Solution | 46.3 |
| Hydrated Silica, Polishing Grade | 17.0 |
| Glycerin | 15.0 |
| Hydrated Silica, Thickening Grade | 11.1 |
| PEG-6-32 | 3.00 |
| Sodium Lauroyl Sarcosinate | 2.10 |
| Flavors | 1.100 |
| Saccharin | 0.100 |
| Colorant | 0.00400 |
| Hydrochloric Acid to make pH 7 | -- |
| Purified Water to make | 100.0 |

Antiplaque Properties of the Compositions

An *in vitro* test for effect of the foregoing compositions against preformed dental plaque was carried out.

*Streptococcus mutans* NCTC 10449, *Streptococcus sanguis* ATCC 10558 and *Actinomyces viscosus* M-100 were used as plaque forming microorganisms and were stored prior to use in a frozen or lyophilized state. Working stock cultures were maintained by twice monthly passage in fluid thioglycollate medium containing 20% (w/v) meat extract and excess calcium carbonate. For plaque formation the medium of Jordan et al. (J. Dent. Res., vol. 39, pp. 116-123, 1960) supplemented with 5% (w/v) sucrose was used. All cultures were adapted to this growth medium by at least one growth cycle prior to use in the tests.

The substrates were cleaned, sterilized ceramic hydroxylapatite (durapatite) slabs approximately 1/4" x 1/2" x 2" in size, which were manipulated during transfer and brushing with sterile clamps (hemostats). Brushing was done with a wetted medium bristle toothbrush and one inch strips of the dentifrice on all sides of the slab for one minute. The slab was then rinsed in running tap water for one minute with manipulation to ensure removal of all visible dentifrice.

Plaques were developed on the slabs by daily passage through the supplemented Jordan et al. medium (20 ml.) in culture tubes. Only the initial culture tube was inoculated with the plaque-forming microorganism (at least 0.3 ml. of a late log-phase culture). Subsequent tubes were not directly inoculated. Thus, only the most adherent microorganisms were carried over, thereby permitting formation of strongly adherent plaques. After three successive daily transfers the slabs were brushed with the dentifrice, rinsed and immersed in fresh growth medium containing a pH indicator (bromocresol purple). The culture tubes were incubated for 24 hours at 37°C. under an anaerobic atmosphere. A once daily brushing

regimen was thus simulated. Alternatively the slabs were brushed twice daily and the incubation periods were 6 and 16 hours in duration. Growth and metabolic activity (acid production) were subsequently assessed, and the treatment and incubation(s) were repeated. The slabs were brushed daily until the cultures failed to yield acid end products and (ideally) yielded viable bacteria.

Metabolic activity (M.A.) was measured 12 and 36 hours after final treatment and scored + (acid production and turbidity increase), $\pm$ (no acid shift but noticeable turbidity increase) or $\mp$ (no acid shift and minimal evidence of turbidity increase). Plaque was measured following staining with erythrosin and scored 0, 1, 2, 3, 4 or Fl (flecks or microcolonies). The following results were obtained from duplicate brushings twice daily for three successive days with only water and no dentifrice, a placebo dentifrice (the composition of Example 2 and 3 wherein the antimicrobial agent was replaced by an equal amount of sorbitol solution) and the compositions of Example 2 and 3.

| Composition | Measurement | Score: S. Mutans 10449 | S. Sanguis 10558 | A. Viscosus M-100 |
|---|---|---|---|---|
| Water | 12-Hr. M.A. | +, + | +, + | ±, ± |
| | 36-Hr. M.A. | +, + | +, + | +, + |
| | Plaque | 3, 3 | 4, 4 | 3, 4 |
| Placebo | 12-Hr. M.A. | +, ± | +, ± | ∓, ∓ |
| | 36-Hr. M.A. | +, + | +, ± | ±, ± |
| | Plaque | 1, 1 | 1, 1 | 1, 1/2 |
| Example 1 | 12-Hr. M.A. | ∓, ∓ | ∓, ∓ | ∓, ∓ |
| | 36-Hr. M.A. | ∓, ± | +, ± | ∓, ∓ |
| | Plaque | Fl, Fl | 1/2, Fl | Fl, Fl |
| Example 2 | 12-Hr. M.A. | ∓, ∓ | ∓, ± | ∓, ∓ |
| | 36-Hr. M.A. | ∓, ∓ | ±, + | ∓, ∓ |
| | Plaque | Fl, Fl | Fl, Fl | Fl, Fl |

<u>Comparative Tests</u>

As stated above previous attempts including CORSODYL™ Dental Gel to combine antiplaque activity, good taste, good foaming ability and good polishing ability in a single dentifrice formulation have been generally unsuccessful. Although CORSODYL™ Dental Gel showed good antiplaque activity against preformed <u>S. mutans</u> plaque on durapatite slabs <u>in vitro</u> after one brushing per day on three successive days, it does not have the other three attributes in good measure when compared with the dentifrice of Example 2.

Comparison of the taste of the dentifrice of Example 2 with that of CORSODYL™ Dental Gel showed that CORSODYL™ Dental Gel had a bitter taste whereas the dentifrice of Example 2 did not have a bitter taste.

The following test was carried out to compare the foaming ability of the dentifrice of Example 2 with that of CORSODYL™ Dental Gel. Each of the test formulations (0.5 g.) was placed in a 50-ml. graduate cylinder together with human salvia which had been passed through a 100-mesh screen (10 ml.) and a toothbrush whose handle had been cut

0144960

off so that it would fit into the cylinder (Abco Dealers Inc. #350100 cut to a length of 1-1/2 inches). More saliva was added to make the total volume 20 ml. The cylinder was securely stoppered and rotated end over end at a constant rate of about 34 revolutions per minute. Foam volumes were read after 25, 50, 75 and 100 revolutions. Seven cylinders were used and mean foam volumes and standard errors were calculated for each formulation. The following results were obtained.

| | Mean Foam Volume (ml.) | |
|---|---|---|
| Number of Revolutions | CORSODYL™ Dental Gel | Dentifrice of Example 2 |
| 25 | 20 ± 0.2 | 22 ± 0.3 |
| 50 | 21 ± 0 | 24 ± 0.3 |
| 75 | 22 ± 0.2 | 25 ± 0.3 |
| 100 | 22 ± 0 | 26 ± 0.3 |
| overall Mean | 21 ± 0.1 | 24 ± 0.3 |

The mean foam volume values for the dentifrice of Example 2 are significantly greater than those of CORSODYL™ Dental Gel ($p = 0.05$).

Due to the presence of the polishing agent the dentifrices of the invention have good polishing ability. CORSODYL™ Dental Gel, on the other hand, appears to contain little or no polishing agent and thus to have little or no polishing ability. This is shown by the fact that CORSODYL™ Dental Gel has a very small residue after combustion (0.05%, 0.06% in two determinations) compared with the dentifrices of Example 2 (19.9%) and Example 3 (19.3%).

Antimicrobial Properties of the Compounds of Formulas IV and V

The utility of the compounds of Formula IV or V as antimicrobial agents has been shown by determining the antibacterial and antifungal properties *in vitro* of a sample of the compound of Example 1 prepared as described above using distilled water instead of deionized water and without heating (m.r. 71.5-73°C.).

A solution of the compound in propylene glycol

(6.4 µM./ml.) was prepared and tested against one strain each of Staphylococcus aureus and Streptococcus pyogenes representing the gram-positive bacteria, Escherichia coli, Klebsiella pneumoniae, Proteus mirabilis, Proteus vulgaris and Pseudomonas aeruginosa representing the gram-negative bacteria, and Aspergillis niger, Candida albicans and Trichophyton mentagrophytes representing the fungi. The cultures of S. aureus, the five gram-negative strains and C. albicans were grown in tryptose phosphate broth. S. pyogenes was grown in brain-heart infusion broth with 10% normal horse serum. These eight cultures were adjusted to an optical density of 0.10 (650 nm., B&L Spectronic 20), then diluted in double-strength culture medium to $2x10^5$ cells/ml. A. niger and T. mentagrophytes were maintained as spore suspensions and diluted in double-strength maltose peptone #3 broth to $2x10^5$ spores/ml. as determined previously by plate count.

Serial twofold dilutions of the propylene glycol solution of the test compound were prepared in sterile distilled water in test tubes. Each dilution was inoculated with an equal volume of each of the diluted microbial cultures and incubated, the bacteria for 18-20 hours at 37°C. and the fungi for five days at 25°C. The lowest concentration of the test compound which prevented visible growth of the microorganism is the minimum inhibitory concentration, whose values are shown in the following table.

| Microorganism | Minimum Inhibitory Concentration (µM./ml.) |
|---|---|
| S. aureus Smith | 1.56 |
| S. pyogenes C203 | 1.56 |
| E. coli Vogel | 1.56 |
| K. pneumoniae 39645 | 6.25 |
| P. mirabilis MGH-1 | 12.5 |
| P. vulgaris 9920 | 3.13 |
| P. aeruginosa MGH-2 | 6.25 |
| A. niger 16404 | 12.5 |
| C. albicans 10231 | 3.13 |
| T. mentagrophytes 9129 | 3.13 |

# CLAIMS

1. A dentifrice composition comprising

A) an effective dental plaque-inhibiting, reducing or preventing amount of the saccharin salt of an amino or quaternary ammonium antimicrobial agent which is

a) a compound having the structural formula

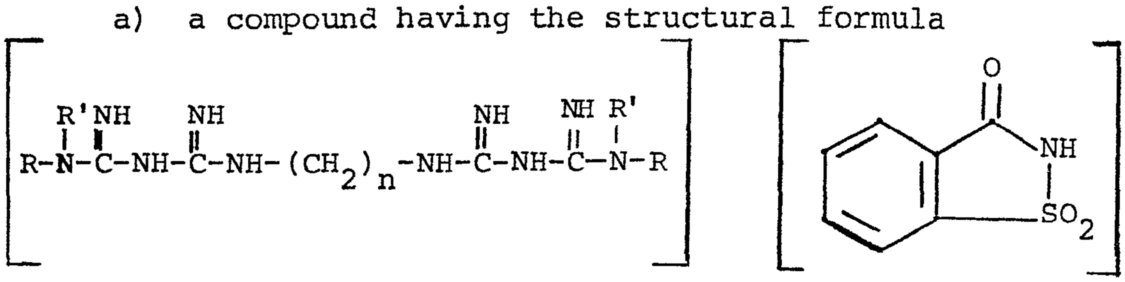

Formula I

wherein R taken alone is phenyl substituted by alkyl, alkoxy, nitro or halo, p-(2,2-dichloro-cyclopropyl) phenyl, alkyl having from 6 to 16 carbon atoms, cycloalkyl or polycyclic alkyl having 5 or more carbon atoms or lower-alkyl-cycloalkyl or cycloalkyl-lower-alkyl having from 1 to 4 carbon atoms in lower alkyl; R' taken alone is hydrogen; R and R' taken together are 3-azabicyclo(3,2,2)nonyl; and n is an integer from 3 to 9;

b) a compound having the structural formula

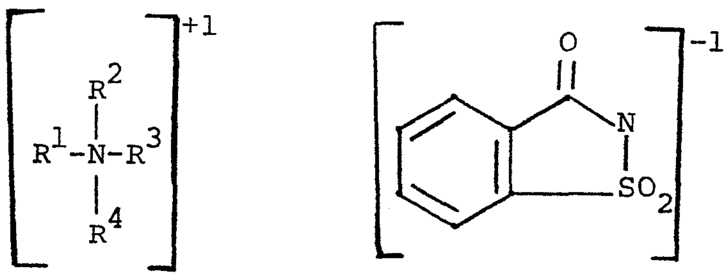

Formula II or

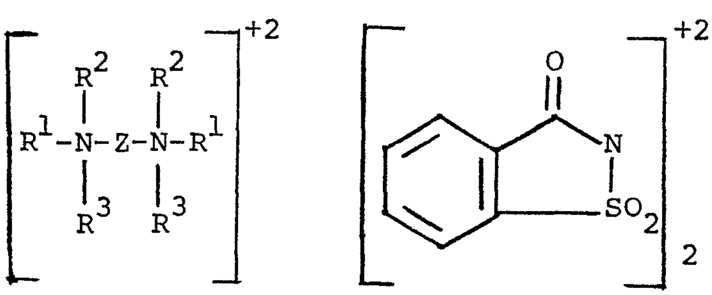

Formula III

wherein $R^1$ is long-chain alkyl or aralkyl; $R^2$ is short-chain alkyl, long-chain alkyl or aralkyl, benzyl or part of an aromatic system or non-aromatic system; $R^3$ and $R^4$ are short-chain alkyl or part of an aromatic ring system or non-aromatic ring system; and Z is a carbon-hydrogen chain; or

c) a compound having the structural formula

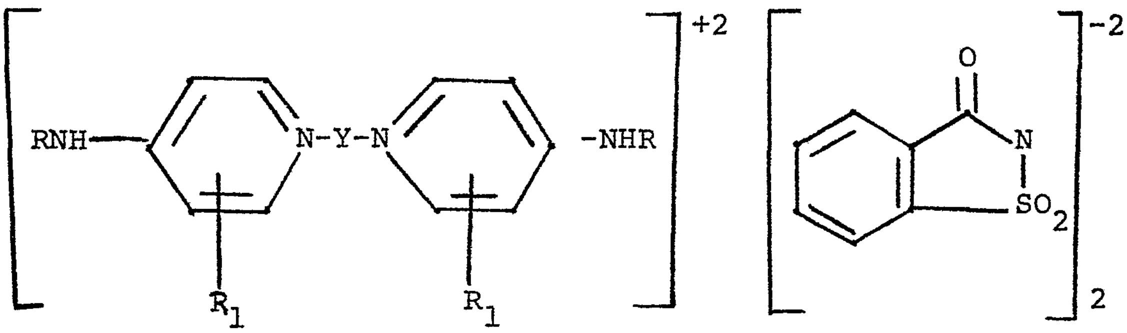

Formula IV

wherein Y is alkylene containing from 4 to 18 carbon atoms and separating the two 4-(R-amino)-1-pyridinium groups by from 4 to 18 carbon atoms; R is the same in both occurrences and is alkyl containing from 6 to 18 carbon atoms, cycloalkyl containing from 5 to 7 carbon atoms, benzyl or phenyl substituted by methylenedioxy or one or two substituents selected from the group consisting of halo, lower-alkyl, lower-alkoxy, nitro, cyano and trifluoromethyl; and $R_1$ is the same in both occurrences and is hydrogen or lower-alkyl;

B) a polishing agent and/or a thickening agent which is silicon dioxide, silicic acid silica, amorphous silica, precipitated silica, hydrated silica, silica hydrate, silica gel, silica xerogel or hydrous silica gel; and

C) a foaming agent which is N-alkanoylsarcosine or N-alkenoylsarcosine or an alkali metal, ammonium or alkanolamine salt thereof, wherein alkanoyl or alkenoyl has from 8 to 18 carbon atoms and alkanol has from 2 to 3 carbon atoms.

2. A dentifrice according to claim 1, wherein the antimicrobial agent is a compound of Formula I.

3. A dentifrice according to claim 2, wherein in the compound of Formula I R is p-chlorophenyl, R' is hydrogen and

n is 6.

4. A dentifrice according to claim 1, wherein the antimicrobial agent is a compound of Formula IV wherein R is octyl, $R_1$ is hydrogen and Y is 1,10-decylene.

5. A dentifrice according to any one of the preceding claims, wherein a polishing agent and a thickening agent are used which are both hydrous silica gels.

6. A dentifrice according to any one of the preceding claims, wherein the foaming agent is sodium lauroyl sarcosinate.

7. A dentifrice according to any one of the preceding claims, wherein there is from about 0.1% to about 10% of the saccharin salt, a total from about 5% to about 50% of the polishing agent and/or thickening agent, and from about 0.1% to about 10% of the foaming agent.

8. A method of inhibiting, reducing or preventing dental plaque which comprises contacting the dental plaque itself or the natural or artificial teeth or oral cavity of a living person with an effective dental plaque-inhibiting, reducing or preventing amount of an antiplaque dentifrice according to any one of the preceding claims.

9. A bis[4-(R-amino)-1-pyridinium]alkane disaccharin salt having the structural formula

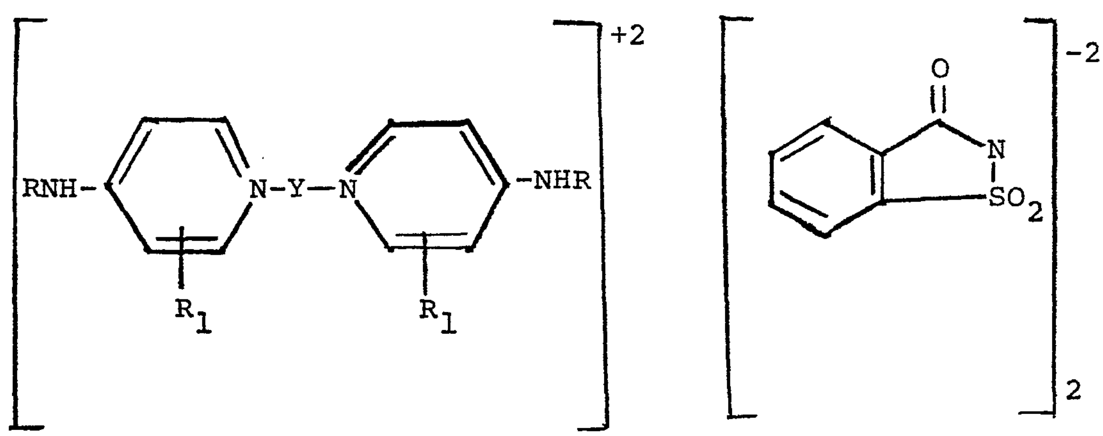

wherein

Y is alkylene containing from 4 to 18 carbon atoms and separating the two 4-(R-amino)-1-pyridinium groups by from 4 to 18 carbon atoms;

R is the same in both occurrences and is alkyl containing from

6 to 18 carbon atoms, cycloalkyl containing from 5 to 7 carbon atoms, benzyl, or phenyl substituted by methylenedioxy or one or two substituents selected from the group consisting of halo, lower-alkyl, lower-alkoxy, nitro, cyano and trifluormethyl; and

$R_1$ is the same in both occurrences and is hydrogen or lower alkyl.

10. N,N'(1,10-Decanediyl-di-1(4H)-pyridin-4-ylidene)-bis[1-octanamine] disaccharin salt.